# EUROPEAN PATENT SPECIFICATION

(11) **EP 3 811 975 B1**
(45) Date of publication and mention of the grant of the patent: **06.08.2025**
(21) Application number: 19826275.0
(22) Date of filing: 24.06.2019
(51) Int. Cl.: A61K 47/04, A61K 9/16, A61K 9/30, A61K 9/36, A61K 47/14, A61K 47/26, A61K 47/32

(54) **COATING COMPOSITION, ORAL SOLID PREPARATION, AND METHOD FOR PRODUCING THE ORAL SOLID PREPARATION**
BESCHICHTUNGSZUSAMMENSETZUNG, ORALE FESTE ZUBEREITUNG UND VERFAHREN ZUR HERSTELLUNG DIESER ORALEN FESTEN ZUBEREITUNG
COMPOSITION DE REVÊTEMENT, PRÉPARATION SOLIDE ORALE ET PROCÉDÉ DE PRODUCTION D'UNE TELLE PRÉPARATION SOLIDE ORALE

(30) Priority: 25.06.2018 JP 2018119468
(43) Date of publication of application: 28.04.2021
(73) Proprietor: Japan Vam & Poval Co., Ltd., Sakai-shi, Osaka 592-8331 (JP)
(72) Inventor: KAWADA, Shotaro, Sakai-shi, Osaka 592-8331 (JP); KAWANISHI, Masatoshi, Sakai-shi, Osaka 592-8331 (JP)
(74) Representative: Kraus & Lederer PartGmbB
(86) International application number: PCT/JP2019/024875
(87) International publication number: WO 2020/004298

(56) References cited:
- WO-A1-2006/062077
- WO-A1-2010/074223
- WO-A1-2011/105539
- WO-A1-2016/108250
- JP-A- 2016 196 439
- US-A1- 2017 354 608
- ISHII, TATSUYA: "A Novel Coating Technology for Imparting Gas Barrier Properties", JOURNAL OF PHARMACEUTICAL MACHINERY AND ENGINEERING, vol. 28, no. 1, March 2019 (2019-03-01), pages 37 - 40, XP009524833, ISSN: 2186-3237
- MANABU SENOO, KAORU TSUJII: "Chemistry of surface activity, and applications. 1st ed.", vol. 51, 25 August 2003, DAINIPPON TOSHO, JP, ISBN: 4-477-00509-1, article "Passage; Surfactant Chemistry and Applications", pages: 1 - 4, XP009524605
- JAPAN OIL CHEMISTRY ASSOCIATION: "Yushi Kagaku Binran. 3rd ed,. [Oleochemistry Handbook Revised 3rd Edition]", 28 February 1990, MARUZEN, ISBN: 9784621034477, article "Passage, Yushi Kagaku Binran. 3rd ed,. [Oleochemistry Handbook Revised 3rd Edition]", pages: 490 - 491, XP009524602
- KOTOR M OHTA , SUGURU TAKAKI , SATOSHI MINAKAMI , YUKI FUJISAKI , YASUHIDE HORIUCHI: "Lumber Technology Formulation and Particle Design Special Feature Coating 3 High Performance Film", PHARM TECH JAPAN , vol. 34, no. 1, 31 December 2017 (2017-12-31), pages 30 - 33, XP009524845, ISSN: 0910-4739

## Description

### TECHNICAL FIELD

The present invention relates to a coating composition used for oral solid preparations, an oral solid preparation coated with the coating composition, and a method for producing the oral solid preparation, etc.

### BACKGROUND ART

Film coating and sugar coating are widely used techniques for coating of oral solid preparations such as drug-containing solid preparations (e.g., tablets etc.) for the purpose of masking of drug-related unpleasant taste, oxygen insulation, moisture-proofing, product appearance improvement, etc.

Film coating is more useful than sugar-coating because it can be performed in a faster and simpler way and can provide a thinner coating film to allow the production of smaller solid preparations, which are easily ingestible oral solid preparations.

For film coating, various polymers including hydroxypropylmethylcellulose (hereinafter abbreviated as HPMC) are used as base materials, and recently, polyvinyl alcohol (hereinafter may be abbreviated as PVA) has drawn attention.

PVA films are excellent in moisture-proofness, and PVA film coating improves the storage stability of solid preparations containing highly hygroscopic components.

However, when PVA is used as a base material of coating compositions, a highly tacky aqueous PVA solution is likely to cause adhesion between solid preparations, adhesion of solid preparations on the inner surface of a coating apparatus, and other undesired events during coating, which hinder the increase of the spray rate, leading to low productivity.

In addition, the moisture-proofness of PVA-based films is higher than that of films using different coating bases, but is reduced under humid conditions.

A known solution to the above problem is to use a coating composition containing PVA in combination with an additive in coating of solid preparations, thereby improving moisture-proofness.

For example, Patent Literature 1 discloses a coating agent comprising PVA, bentonite or aluminum magnesium silicate, and a sugar alcohol derivative-based surfactant. Patent Literature 1 states that the moisture-proofness of a coat formed from the coating agent is high even under humid conditions. Also stated is that the mass ratio of PVA and bentonite or aluminum magnesium silicate in the coating agent is 2: 8 to 5:5.

However, Patent Literature 1 discloses no specific combination of PVA, a swellable clay mineral, a sugar alcohol derivative-based surfactant, and talc.

WO 2010/074223 A1 discloses a coating material for a solid formulation comprising a high hydrogen-bonding resin and a swelling agent, and a solid formulation using the same.

WO 2016/108250 A1 discloses a film coating composition for substrates like pharmaceutical tablets, nutritional supplements, food products, comprising polyvinyl alcohol (PVA) in combination with sugar and a plasticizer.

WO 2006/062077 A1 discloses a film coating composition containing a polyvinyl alcohol and talc, wherein the talc content is 50-86% by mass of the film coating composition. Furthermore disclosed in WO 2006/062077 A1 are a coating film made of this film coating composition and a tablet having such a coating film.

### CITATION LIST

### Patent Literature

Patent Literature 1: Japanese Patent No. 5609639

### SUMMARY OF INVENTION

### TECHNICAL PROBLEM

An object of the present invention is to provide a novel coating composition for oral solid preparations.

Another object of the present invention is to provide a coating composition for oral solid preparations which coating composition forms a highly moisture-proof coating film.

Another object of the present invention is to provide a coating composition for oral solid preparations which coating composition is less likely to develop tackiness during coating.

Another object of the present invention is to provide a coating composition for oral solid preparations which coating composition provides oral solid preparations with fast-dissolving property.

Another object of the present invention is to provide a coating composition for oral solid preparations which coating composition is practical and applicable to common oral solid preparations.

Yet another object of the present invention is to provide an oral solid preparation coated with the above coating composition.

Yet another object of the present invention is to provide a method for producing an oral solid preparation coated with the above coating composition.

### SOLUTION TO PROBLEM

The coating agent or composition of Patent Literature 1 has a high proportion of clay minerals such as bentonite or aluminum magnesium silicate so that it can provide highly moisture-proof coating. However, a greater amount of the clay mineral increases the viscosity of a coating composition solution and thus necessitates reduction in the solid content in the coating composition solution, resulting in a prolonged time to achieve a predetermined coating weight gain and considerably low productivity. The present inventors considered this as a problem to be solved. The present inventors also noted the dissolution rate of the component in solid preparations coated with the coating agent described in Patent Literature 1 and found that it tends to be lower than the dissolution rate of the component in ordinary fast-dissolving tablets.

The present inventors conducted extensive research to solve the above-mentioned problems. As a result, the present inventors found that a coating composition which comprises a PVA polymer (a), a water-swellable clay mineral (b), and a fatty acid ester of a polyol having 3 or more hydroxy groups (c) and has a specific mass ratio of the PVA-based polymer (a) and the swellable clay mineral (b) (the PVA-based polymer (a) and the water-swellable clay mineral (b) are present at a mass ratio of 98:2 to 65:35 in the coating composition) can provide excellent moisture-proof coating of solid preparations.

As described above, Patent Literature 1 states that, in the coating agent comprising PVA and bentonite or aluminum magnesium silicate, the amount of bentonite or aluminum magnesium silicate is desirably equal to or greater than the amount of PVA on a mass basis. In contrast, surprisingly, the present inventors conceived an idea that the amount of the swellable clay mineral (b) is equal to or smaller than the amount of the PVA-based polymer (a) on a mass basis. Such a coating composition having a smaller amount of the swellable clay mineral (b) can provide highly moisture-proof coating, which is a very unexpected effect.

In addition, the present inventors found that a coating composition comprising a PVA-based polymer (a), a water-swellable clay mineral (b), a fatty acid ester of a polyol having 3 or more hydroxy groups (c), and talc (d) also can provide excellent moisture-proof coating and other advantages. The present inventors further conducted a great deal of examination and then completed the present invention.

The present invention is set out in the appended set of claims. That is, the present invention relates to the following.

A coating composition for oral solid preparations, the coating composition comprising a polyvinyl alcohol-based polymer (a), a water-swellable clay mineral (b), and a fatty acid ester of a polyol having 3 or more hydroxy groups (c), wherein the polyvinyl alcohol-based polymer (a) and the water-swellable clay mineral (b) are present at a mass ratio of 98:2 to 65:35 in the coating composition.

A coating composition for oral solid preparations, the coating composition comprising a polyvinyl alcohol-based polymer (a), a water-swellable clay mineral (b), a fatty acid ester of a polyol having 3 or more hydroxy groups (c), and talc (d) .

An oral solid preparation coated with the coating composition of the present invention.

A method for producing an oral solid preparation, the method comprising the step of applying or spraying an aqueous and/or water-based solution containing the coating composition of the present invention to a solid preparation to cover a surface of the solid preparation with the coating composition.

The use of a composition for coating an oral solid preparation, the composition comprising a polyvinyl alcohol-based polymer (a), a water-swellable clay mineral (b), and a fatty acid ester of a polyol having 3 or more hydroxy groups (c), wherein the polyvinyl alcohol-based polymer (a) and the water-swellable clay mineral (b) are present at a mass ratio of 98:2 to 65:35 in the composition.

The use of a composition for coating an oral solid preparation, the composition comprising a polyvinyl alcohol-based polymer (a), a water-swellable clay mineral (b), a fatty acid ester of a polyol having 3 or more hydroxy groups (c), and talc (d).

Preferred embodiments of the present invention are also set out in the appended set of claims and will be described in the DESCRIPTION OF EMBODIMENTS below.

### ADVANTAGEOUS EFFECTS OF INVENTION

The present invention provides a novel coating composition for oral solid preparations. The coating composition comprises a PVA-based polymer (a) and a water-swellable clay mineral (b) at a specific mass ratio and further comprises a fatty acid ester of a polyol having 3 or more hydroxy groups (c), and forms a coating film having high moisture-proofness (particularly, high moisture-proofness even under humid conditions).

The present invention also includes a coating composition for oral solid preparations which coating composition comprises a PVA-based polymer (a), a water-swellable clay mineral (b), a fatty acid ester of a polyol having 3 or more hydroxy groups (c), and talc (d). Such a coating composition also forms a coating film having high moisture-proofness.

When the coating composition of the present invention is used for coating of solid preparations, adhesion between solid preparations is less likely to occur, leading to reduction in coating time and improvement in coating productivity.

The coating composition of the present invention can provide oral solid preparations with fast-dissolving property.

The coating composition of the present invention is practical and applicable to common oral solid preparations.

In addition, the present invention provides an oral solid preparation coated with the above coating composition.

Further, the present invention provides a method for producing an oral solid preparation coated with the above coating composition. In this method, less adhesion between solid preparations during coating results in efficient coating.

### DESCRIPTION OF EMBODIMENTS

### Coating composition for oral solid preparations

The coating composition of the present invention for oral solid preparations comprises a polyvinyl alcohol-based polymer (a), a water-swellable clay mineral (b), and a fatty acid ester of a polyol having 3 or more hydroxy groups (c). This coating composition comprises the polyvinyl alcohol-based polymer (a) and the water-swellable clay mineral (b) at a specific mass ratio as described later.

The present invention also includes a coating composition for oral solid preparations which coating composition comprises a polyvinyl alcohol-based polymer (a), a water-swellable clay mineral (b), a fatty acid ester of a polyol having 3 or more hydroxy groups (c), and talc (d).

The components of the coating composition of the present invention usually refer to the components other than a solvent (e.g., a liquid component such as water or an organic solvent) in a coating composition solution used in coating. When the coating composition is described below, the mass ratio and mass percentage of each component in the coating composition usually refer to a proportion relative to the components other than a solvent.

### Polyvinyl alcohol-based polymer (a)

The polyvinyl alcohol-based polymer (may be referred to as a PVA-based polymer, PVA, etc.) (a) is usually a saponified product of a vinyl ester-based polymer (a polymer composed of a vinyl ester as at least a polymerization component).

The vinyl ester (vinyl ester monomer) is not particularly limited, and examples include fatty acid vinyl esters [e.g., C₁₋₂₀ fatty acid vinyl esters (e.g., C₁₋₁₆ alkanoic acid vinyl esters) such as vinyl formate, vinyl acetate, vinyl propionate, vinyl butyrate, vinyl caprylate, vinyl versatate, and vinyl monochloroacetate], and aromatic carboxylic acid vinyl esters [e.g., vinyl arenecarboxylates (e.g., C₇₋₁₂ arene carboxylic acid vinyl esters) such as vinyl benzoate].

One kind of vinyl ester or a combination of two or more kinds of vinyl esters may be used.

The vinyl ester preferably at least contains a fatty acid vinyl ester (e.g., C₁₋₁₀ alkanoic acid vinyl esters etc., such as vinyl formate, vinyl acetate, vinyl propionate, and vinyl butyrate). Particularly, vinyl acetate is preferred from industrial or other viewpoints.

The vinyl ester-based polymer has a vinyl ester unit, and if necessary, may have an additional monomer unit (a monomer capable of copolymerizing with vinyl esters) (in other words, the vinyl ester-based polymer may be modified with an additional monomer).

The additional monomer is not particularly limited, and examples include, but are not limited to, alpha-olefins (e.g., ethylene, propylene, etc.), (meth)acrylic acid esters [e.g., (meth) acrylic acid alkyl esters such as methyl (meth) acrylate, ethyl (meth)acrylate, butyl (meth)acrylate, and 2-ethylhexyl (meth)acrylate], unsaturated amides [e.g., (meth)acrylamide, diacetone acrylamide, N-methylolacrylamide, etc.], unsaturated acids {e.g., unsaturated acids [e.g., (meth) acrylic acid, crotonic acid, maleic acid, itaconic acid, fumaric acid, etc.], unsaturated acid esters [unsaturated acid esters other than (meth)acrylic acid esters, e.g., alkyl (methyl, ethyl, propyl, etc.) esters etc.], unsaturated acid anhydrides (maleic anhydride etc.), salts of unsaturated acids [e.g., alkali metal salts (e.g., sodium salts, potassium salts, etc.), ammonium salts, etc.], etc.}, glycidyl group-containing monomers [e.g., allyl glycidyl ethers, glycidyl (meth)acrylate, etc.], sulfonic group-containing monomers (e.g., 2-acrylamide-2-methylpropane sulfonic acid, salts thereof, etc.), phosphate group-containing monomers [e.g., acid phosphoxy ethyl (meth)acrylate, acid phosphoxy propyl (meth) acrylate, etc.], vinyl ethers (e.g., alkyl vinyl ethers), allyl alcohols, etc.

One kind of additional monomer or a combination of two or more kinds of additional monomers may be used.

In the PVA-based polymer (a), some vinyl alcohol units may be modified by a reaction, such as acetalization, etherification, acetoacetylization, or cationization.

One kind of PVA-based polymer (a) or a combination of two or more kinds of PVA-based polymers (a) may be used.

The PVA-based polymer (a) may be a commercial product.

The method for producing the PVA-based polymer (a) is not particularly limited, and known methods, for example, saponification of a vinyl ester-based polymer, are may be used.

The polymerization method for the vinyl ester-based polymer is not particularly limited, and examples include known polymerization methods such as block polymerization, solution polymerization, suspension polymerization, and emulsion polymerization. Among them, solution polymerization (e.g., solution polymerization using methanol as a solvent) is industrially preferred.

In the solution polymerization, known initiators such as peroxide initiators and azo initiators can be used, and the polymerization degree of the vinyl ester-based polymer can be adjusted by varying the feed ratio of the vinyl ester monomer and a solvent and the polymerization yield.

The method for saponifying the vinyl ester-based polymer can be a conventional saponification method using an alkaline or acid catalyst. In particular, industrially preferred is alcoholysis, which is performed by adding an alkali such as sodium hydroxide to a solution of the vinyl ester-based polymer in methanol or in a mixed solvent of methanol, water, methyl acetate, etc. and stirring the mixture.

After that, optionally, the obtained mass product, gelled product, or granular product is pulverized, and if needed, the alkali is neutralized; then the solid matter is separated from the liquid matter and dried to yield a PVA-based polymer.

The saponification value of the PVA-based polymer (a) is not particularly limited and is preferably within the standard of the saponification value of PVA described in the following three official compendiums: the Japan Pharmaceutical Excipient Standards, the United States Pharmacopeia, and the European Pharmacopoeia. In addition, the average saponification value of the PVA-based polymer is, for example, preferably 74.0 mol% to 89.0 mol% (e.g., 80.0 to 89.0 mol% etc.) and particularly preferably 85.0 mol % to 89.0 mol% for fast dissolution in a living body.

When the average saponification value is 85.0 mol% or more, the PVA-based polymer (a) can be used as a material of pharmaceutical preparations adapted for global markets. In addition, the proportion of hydrophobic groups in such a PVA-based polymer (a) is low enough so that the PVA-based polymer (a) is highly hydrophilic, less likely to precipitate at high temperature in preparing an aqueous solution, and easy to handle. That is why the lower limit specified above is particularly preferred.

Similarly, when the average saponification value is 89.0 mol% or less, the PVA-based polymer (a) can be used as a material of pharmaceutical preparations adapted for global markets. In addition, the proportion of hydroxyl groups in PVA is not so high as to result in reduced water solubility due to high crystallinity or reduced dissolution rate of oral solid preparations coated with the coating composition of the present invention. That is why the upper limit specified above is particularly preferred.

The method for measuring the average saponification value of PVA is not particularly limited, and for example, the method for measuring the saponification value specified in JIS K6726 can be used.

The polymerization degree of the PVA-based polymer (a) is not particularly limited, and the viscosity of its 4% by mass aqueous solution is, for example, preferably 2.0 to 10.0 mPa·s (e.g., 3.0 to 9.0 mPa·s etc.) and more preferably 3.0 to 7.0 mPa·s.

When the viscosity of the 4% by mass aqueous solution is 2.0 mPa·s or more, a high-strength coat layer can be formed on the surface of solid preparations after coating. That is why the lower limit specified above is preferred. When the viscosity of the 4% by mass aqueous solution is 10 mPa·s or less, the viscosity is low enough to allow a high-rate spray during coating, resulting in high productivity. That is why the upper limit specified above is preferred.

The method for measuring the viscosity of the 4% by mass aqueous solution is not particularly limited, and for example, the method specified in JIS K6726 can be used for viscosity measurement.

### Water-swellable clay mineral (b)

The water-swellable clay mineral usually means a clay mineral that absorbs water to swell.

The water-swellable clay mineral (b) is, for example, preferably a negatively-charged one, and may be a layered silicate mineral or the like.

The term "negatively-charged" usually means a state in which the water-swellable clay mineral (b) has cation exchange property. The amount of negative charge can be expressed as cation exchange capacity (CEC). The unit of the cation exchange capacity is mg equivalent/100 g (usually expressed as meq/100 g) and can generally be expressed as the number of equivalents corresponding to the molar concentration of monovalent ions.

The cation exchange capacity of the water-swellable clay mineral (b) is not particularly limited and may be, for example, about 60 to 150 meq/100 g.

The water-swellable clay mineral (b) is not particularly limited, and examples include smectites (e.g., montmorillonite, saponite, beidellite, hectorite, stevensite, sauconite, nontronite, etc.), aluminum magnesium silicate, etc.

Among them, montmorillonite, aluminum magnesium silicate, etc. are preferred.

The water-swellable clay mineral (b) may be a water-swellable clay containing the water-swellable clay mineral (b) and is, for example, preferably a water-swellable clay mainly containing smectite, and more preferably a water-swellable clay mainly containing montmorillonite. In particular, bentonite is preferred.

The water-swellable clay mineral (b) may be a synthetic or natural one.

One kind of water-swellable clay mineral (b) or a combination of two or more kinds of water-swellable clay minerals (b) may be used.

### Fatty acid ester of a polyol having 3 or more hydroxy groups (c)

In the fatty acid ester of a polyol having 3 or more hydroxy groups (c) (hereinafter may be referred to simply as a polyol fatty acid ester (c)), the number of hydroxy groups is 3 or more, and for example, but not particularly limited to, 3 to 10 (e.g., 3 to 8 etc.).

Examples of the polyol having 3 or more hydroxy groups include sugars [e.g., monosaccharides (e.g., glucose, galactose, mannose, fructose, etc.), disaccharides (e.g., saccharose, maltose, lactose, trehalose, etc.)], sugar alcohols (e.g., erythritol, lactitol, maltitol, mannitol, sorbitol, xylitol, inositol, sorbitan, etc.), alkane polyols [e.g., alkane triols (e.g., C₃₋₁₀ alkane triols, such as glycerin, butanetriol, and hexanetriol, preferably C₃₋₆ alkane triols)], polyalkane polyols {e.g., polyalkane triols [e.g., polyglycerin (e.g., diglycerin, triglycerin) and other polyalkane triols, preferably di- to tri-C₃₋₆ alkane triols]}, etc.

In addition, the polyol having 3 or more hydroxy groups may be in the form of an adduct with an oxyalkylene (e.g., a polyoxyalkylene adduct). Examples of such an adduct include polyoxyalkylene adducts of the exemplary compounds listed above [e.g., polyoxyalkylene sorbitan (e.g., polyoxyethylene sorbitan etc.), polyoxyalkylene glycerin (e.g., polyoxyethylene glycerin etc.), etc.].

Among these polyols having 3 or more hydroxy groups, sugars, sugar alcohols, etc. are preferred, and saccharose, sorbitan, etc. are particularly preferred.

One kind of polyol having 3 or more hydroxy groups or a combination of two or more kinds of polyols having 3 or more hydroxy groups may be used.

In the polyol fatty acid ester (c), a fatty acid ester unit is a hydrophobic group and may be saturated or unsaturated and straight-chained or branched-chained.

The fatty acid ester unit preferably has 12 to 22 carbon atoms.

When the number of carbon atoms is 12 or more, the proportion of hydrophobic groups in the molecule is high enough to provide excellent moisture-proofing in combination with PVA. That is why the lower limit specified above is preferred. When the number of carbon atoms is 22 or less, the proportion of hydrophobic groups in the molecule is low enough to make it easy to dissolve and/or disperse the coating composition in water to prepare a uniform aqueous solution. That is why the upper limit specified above is preferred.

Examples of the fatty acid constituting the fatty acid ester unit include C₁₂₋₂₂ fatty acids {e.g., C₁₂₋₂₂ saturated fatty acids [e.g., C₁₂₋₂₂ alkanoic acids, such as lauric acid, myristic acid, pentadecyl acid, palmitic acid, heptadecanoic acid, and stearic acid], C₁₂₋₂₂ unsaturated fatty acids [e.g., C₁₂₋₂₂ mono-unsaturated fatty acids (e.g., C₁₂₋₂₂ alkenoic acids, such as oleic acid, myristoleic acid, and palmitoleic acid), C₁₂₋₂₂ di- to hexa-unsaturated fatty acids (e.g., C₁₂₋₂₂ alkadienoic acids, such as linoleic acid, C₁₂₋₂₂ alkatrienoic acids, such as linolenic acid), etc.]} etc.

One kind of fatty acid or a combination of two or more kinds of fatty acids may be used.

The polyol fatty acid ester (c) may be, for example, a monoester, a polyester (e.g., a di- to hexa-ester), or the like. Preferably, the polyol fatty acid ester (c) has a high proportion of monoesters (e.g., the monoester content is 30 to 90%, 50 to 90%, etc.).

The hydrophilic lipophilic balance (HLB) value of the polyol fatty acid ester (c) is not particularly limited and is, for example, preferably 4 or more (e.g., 4 to 20, 4 to 18, 10 to 18, 12 to 18, etc.). When the HLB value is 4 or more, the proportion of monoesters is high enough to make it easy to dissolve and/or disperse the coating composition in water to prepare a uniform aqueous solution. That is why the lower limit specified above is preferred.

The method for calculating the HLB value is not particularly limited, and for example, the HLB value may be calculated according to the Atlas HLB system (HLB value = 20 (1-S/A) wherein S is a saponification value and A is an acid value of a fatty acid).

One kind of polyol fatty acid ester (c) or a combination of two or more kinds of polyol fatty acid esters (c) may be used.

### Talc (d)

The talc (d) is not particularly limited and is, for example, a talc generally used as an additive for pharmaceutical and food products, preferably a talc specified in the Japanese Pharmacopoeia.

### Additional components

The coating composition for oral solid preparations may comprise an additional component other than the polyvinyl alcohol-based polymer (a), the water-swellable clay mineral (b), the fatty acid ester of a polyol having 3 or more hydroxy groups (c), and talc (d).

The additional component is not particularly limited, and examples include various additives such as colorants [pigments (e.g., titanium oxide, aluminum lake, iron oxide, etc.), natural colorants, etc.], drugs usually used for pharmaceutical preparations, lubricants (e.g., magnesium stearate, calcium stearate, stearic acid, etc.), polymers other than the PVA-based polymer (a) (e.g., hydroxypropylmethyl cellulose, hydroxypropyl cellulose, etc.), surfactants other than the polyol fatty acid ester (c), sweeteners, coating materials, defoamers, pH adjusters, etc.

Among them, colorants are preferred.

Among colorants, preferred is titanium oxide. One of the reasons is that it can provide hue improvement on the surface of solid preparations after coating.

One kind of additional component or a combination of two or more kinds of additional components may be used.

### Embodiments of coating composition for oral solid preparations

In the composition, the proportion of the PVA-based polymer (a) (the proportion of the PVA-based polymer (a) in the whole composition) is not particularly limited and may be, for example, about 25 to 90% by mass (e.g., 30 to 80% by mass), preferably about 35 to 70% by mass, and more preferably about 40 to 60% by mass.

In the composition, the proportion of the water-swellable clay mineral (b) (the proportion of the water-swellable clay mineral (b) in the whole composition) is not particularly limited and may be, for example, about 1 to 30% by mass, preferably about 3 to 20% by mass, and more preferably about 7 to 15% by mass.

In the composition, the proportion of the polyol fatty acid ester (c) (the proportion of the polyol fatty acid ester (c) in the whole composition) is not particularly limited and may be, for example, about 2 to 45% by mass, preferably about 5 to 30% by mass, and more preferably about 5 to 20% by mass (e.g., 7 to 15% by mass).

In the composition, the proportion of the talc (d) (the proportion of the talc (d) in the whole composition) is not particularly limited and may be, for example, preferably about 5 to 50% by mass (e.g., 10 to 50% by mass, 20 to 50% by mass, etc.) and more preferably about 30 to 50% by mass.

When the proportion of the talc is 50% by mass or less, the coat layer after coating hardly becomes brittle. That is why the upper limit specified above is preferred.

In the composition comprising the additional component, the proportion of the additional component (the proportion of the additional component in the whole composition) is not particularly limited and may be, for example, about 1 to 50% by mass, preferably about 2 to 30% by mass, and more preferably about 5 to 20% by mass.

In the composition, the ratio of the PVA-based polymer (a) and the water-swellable clay mineral (b) is not particularly limited, and the ratio (a): (b) (mass ratio) is, for example, preferably 98:2 to 65:35 (e.g., 85:15 to 65:35, etc.) and more preferably 80:20 to 70:30.

When the proportion of (b) is higher than 98:2 (mass ratio (a): (b)), a sufficiently moisture-proof coat layer can be formed from the coating composition. That is why the lower limit specified above is preferred.

When the proportion of (b) is lower than 65:35 (mass ratio (a): (b)), a solution of the coating composition dissolved and/or dispersed in water has reduced viscosity, resulting in a shortened coating time. That is why the upper limit specified above is preferred.

In the composition, the ratio of the PVA-based polymer (a) and the polyol fatty acid ester (c) is not particularly limited, and the ratio (a): (c) (mass ratio) is, for example, preferably 95:5 to 50:50, more preferably 90:10 to 60:40, and particularly preferably 80:20 to 70:30.

When the proportion of (c) is higher than 95:5 (mass ratio (a) : (c)), the tackiness of PVA is reduced enough to result in less adhesion between solid preparations during coating. That is why the lower limit specified above is preferred.

When the proportion of (c) is lower than 50:50 (mass ratio (a) : (c), a high-strength coat layer can be formed. That is why the upper limit specified above is preferred.

In the composition, the ratio of the PVA-based polymer (a) and the talc (d) is not particularly limited, and the ratio (a) : (d) (mass ratio) may be, for example, 95:5 to 30:70 (e.g., 90:10 to 30:70), preferably 80:20 to 40:60, and more preferably 70:30 to 50:50.

In the composition, the ratio of the water-swellable clay mineral (b) and the talc (d) is not particularly limited, and the ratio (b) : (d) (mass ratio) may be, for example, 98:2 to 10:90 (e.g., 90:10 to 10:90), preferably 70:30 to 30:70, and more preferably 60:40 to 40:60.

In the composition comprising the additional component, the ratio of the additional component to the PVA-based polymer (a) is not particularly limited, and for example, the amount of the additional component is preferably 1 to 100 parts by mass (e.g., 1 to 80 parts by mass, 1 to 60 parts by mass, etc.) and more preferably 1 to 50 parts by mass relative to 100 parts by mass of the PVA-based polymer (a).

The form of the coating composition for oral solid preparations in use is not particularly limited, and the coating composition may be prepared in a liquid form (e.g., an aqueous solution, a water-based solution, etc.) by dissolution or dispersion in a solvent (e.g., water, an organic solvent, a mixed solvent of water and an organic solvent, etc.).

The organic solvent is not particularly limited, and examples include alcohols (e.g., ethanol etc.) etc.

One kind of solvent or a combination of two or more kinds of solvents may be used.

The solvent is preferably only water in view of environmental impact, the influence of the residual organic solvent in solid preparations, and other perspectives.

For the dissolution or dispersion of the coating composition in the solvent, known methods may be used. For example, the coating composition is dispersed in a solvent (a solvent heated if needed, e.g., water, hot water, etc.), and the mixture is stirred with heating or at ordinary temperature to prepare a coating composition solution.

Alternatively, the PVA-based polymer (a), the water-swellable clay mineral (b), and the fatty acid ester of a polyol having 3 or more hydroxy groups (c) may be separately added to solvents (solvents heated if needed, e.g., water, hot water, etc.) to prepare a coating composition solution.

In the case where the coating composition for oral solid preparations is used in a liquid form, the solid content in the liquid may be, for example, about 1 to 50% by mass and preferably about 1 to 30% by mass.

### Oral solid preparation

The present invention also includes an oral solid preparation coated with the coating composition of the present invention.

The oral solid preparation comprises, for example, a solid preparation and a coat (coat layer) covering the solid preparation, and the coat at least comprises the coating composition.

Examples of the form of the solid preparation include tablets, granules, fine granules, etc. Among them, tablets are preferred.

The solid preparation may be, for example, a pharmaceutical product, a quasi-pharmaceutical product, a food product, or the like.

Examples of the solid preparation include a pharmaceutical preparation, a quasi-pharmaceutical preparation, a health food (e.g., a food for special dietary uses, a food for specified health uses, a food with nutrient function claims, a functional food, a dietary supplement, a health supplement, a nutritionally fortified food, etc.), etc.

The component of the solid preparation can be appropriately determined based on, for example, the embodiment or application of the solid preparation. The component of the solid preparation may be, for example, an active ingredient, a nutrient, or the like.

The solid preparation usually may comprise an additive that is commonly used in this field (e.g., a filler, a binder, a disintegrant, a lubricant, an antiagglomerant, a solubilizer for pharmaceutical compounds, etc.).

Examples of the filler include saccharides, such as sucrose, lactose, mannitol, and glucose, starch, crystalline cellulose, calcium phosphate, calcium sulfate, etc.

Examples of the binder include PVA, polyacrylic acid, polymethacrylic acid, polyvinyl pyrrolidone, glucose, sucrose, lactose, maltose, dextrin, sorbitol, mannitol, hydroxyethyl cellulose, HPMC, hydroxypropyl cellulose, macrogols, gum arabic, gelatin, agar, starch, etc.

Examples of the disintegrant include low-substituted hydroxypropyl cellulose, carmellose or a salt thereof, croscarmellose sodium, sodium carboxymethyl starch, cross-linked polyvinylpyrrolidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer for pharmaceutical compounds include organic acids, such as fumaric acid, succinic acid, malic acid, and adipic acid, etc.

One of these additives or two or more of them may be used.

The amount of the additive can be appropriately determined based on, for example, the kind(s) of the component(s) of the solid preparation.

The oral solid preparation may have a plurality of coat layers.

For example, the oral solid preparation may have an undercoating layer under the coat layer formed from the coating composition of the present invention and may have an overcoating layer over the coat layer.

The undercoating layer and the overcoating layer may be formed by, for example, coating with a composition containing, as a component, a polymer (e.g., HPMC etc.) commonly used for coating of pharmaceutical preparations.

### Method for producing oral solid preparation

The present invention also includes a method for producing an oral solid preparation, which method comprises the step of applying or spraying an aqueous and/or water-based solution containing the coating composition of the present invention to a solid preparation to cover the surface of the solid preparation with the coating composition.

The method for covering the surface of the solid preparation with the coating composition is not particularly limited, and known types of coating, for example, film coating etc. may be used.

The coating technique may be, for example, spray coating.

The coating apparatus used may be, for example, a pan coater, a drum coater, or the like. These apparatuses may be equipped with an air spray, an airless spray, or other types of spray devices.

In one embodiment, the surface of the solid preparation can be covered with the coating composition as follows. A coating composition solution is prepared by dissolving or dispersing the coating composition of the present invention, which optionally contains an additive, in a solvent [e.g., water, an organic solvent (e.g., alcohols such as ethanol etc.), a mixed solvent of water and an organic solvent, etc.], and the solution is sprayed or applied to a solid preparation using the above-mentioned coating apparatus in parallel with drying to cover the surface of the solid preparation.

The coating weight of the coating composition for coating of the surface of the solid preparation varies with the type, form, size, and surface condition of the solid preparation, the properties of the components and additives contained in the solid preparation, and other factors. For example, the coating weight is preferably 1 to 10% by mass, more preferably 1 to 7% by mass, and particularly preferably 2 to 6% by mass relative to the total weight of the solid preparation. When the coating weight is within this range, perfect coating is achieved, and sufficient moisture-proofing, oxygen barrier, and odor masking are provided. In addition, the time for coating is shortened. That is why the range specified above is preferred.

### EXAMPLES

Hereinafter, the present invention will be described in more detail by examples, but the present invention is not limited thereto.

In the following Examples and Comparative Examples, "percentage (%) " and "part" are on a mass basis unless otherwise specified.

### Coating conditions

Apparatus: HICOATER (HC-FZ-Labo, manufactured by Freund Corporation)
Tablet feed: 1000 g
Inlet air temperature: 70 to 80°C
Outlet air temperature: 44 to 52°C
Inlet air flow: 0.6 m³/min
Number of spray guns: 1
Spray gun Spray air flow (atomized air): 30 L/min
Spray gun Spray air flow (patterned air): 9 L/min
Spray rate: adjusted based on the discharge flow from the tube pump
Pan rotation speed: 18 rpm

### Evaluation of coating time

In a coating test, a coating composition solution was initially sprayed at a spray rate of 2.0 g/min, and when no adhesion between tablets or between tablets and the pan occurred, the spray rate was gradually increased until the adhesion between tablets or between tablets and the pan occurred. Once the adhesion occurred, the spray rate was decreased to the extent that no adhesion between tablets or between tablets and the pan occurred. The spray rate was fixed at this level, and spray coating was continued for 10 minutes. When no adhesion occurred during the 10 minutes, this spray rate was regarded as the maximum spray rate. When the adhesion between tablets or between tablets and the pan occurred at the initial spray rate of 2.0 g/min, the spray rate was gradually decreased to the extent that no adhesion between tablets or between tablets and the pan occurred. The spray rate was fixed at this level, and spray coating was continued for 10 minutes. When no adhesion occurred during the 10 minutes, this spray rate was regarded as the maximum spray rate. In addition, the minimum coating time required to achieve a coating mass gain of 3% in terms of the solid content of the tablet was calculated from the maximum spray rate.

### Evaluation of water vapor transmission rate

A solution or dispersion of the coating composition at a solid content of 6% by mass was cast on a PET sheet, dried in a constant-temperature-and-humidity chamber at 20°C and RH65% to give an about 100-µm-thick film. The water vapor transmission rate of the obtained film was measured using an L80-5000 water vapor transmission analyzer (manufactured by Systech Instruments) according to the method specified in JIS K7129 at 25°C and a relative humidity difference of 75%.

### Evaluation of dissolution rate

A dissolution test was performed under the conditions described below, and the dissolution rate in 10 minutes was measured and calculated.
Test method: the Japanese Pharmacopoeia dissolution test Method 2 (paddle method)
Test fluid: 900 mL of distilled water
Paddle rotation speed: 50 rpm
Samples: tablets shown in Examples and Comparative Examples
Detection wavelength: UV 444 nm

### Example 1

12.0 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 3.0 parts by mass of bentonite (manufactured by KUNIMINE INDUSTRIES CO., LTD., KUNIPIA F), 3.0 parts by mass of sucrose fatty acid ester (manufactured by Mitsubishi-Chemical Foods Corporation, O-1570, number of carbon atoms per fatty acid ester unit: 18, HLB value: 15), and 12.0 parts by mass of talc (manufactured by Nippon Talc Co., Ltd.) were added to 270.0 parts by mass of purified water with stirring. The mixture was stirred for 1.5 hour to prepare a coating composition solution (concentration in water: 10% by mass). The coating test was performed using this coating composition solution and a plain tablet composed mainly of lactose and cornstarch to evaluate the maximum spray rate, the minimum coating time, and the water vapor transmission rate of the coating composition.

The maximum spray rate in Example 1 was 5.5 g/min, and the coating time required to achieve a coating mass gain of 3% was 55 minutes. The dissolution rate in 10 minutes was 100%, and the water vapor transmission rate was 53 g/m²·day. The results are shown in Table 2.

### Examples 2 to 8

The coating test was performed in the same manner as in Example 1 except for using the coating composition consisting of the components described in Table 1 to evaluate the maximum spray rate, the minimum coating time, and the water vapor transmission rate of the coating composition. The results are shown in Table 2.

### Comparative Example 1

12.0 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s) and 18.0 parts by mass of bentonite (manufactured by KUNIMINE INDUSTRIES CO., LTD., KUNIPIA F) were added to 970.0 parts by mass of purified water with stirring using a homogenizer. The mixture was stirred for 1 hour to prepare a coating composition solution (concentration in water: 3% by mass). The coating test was performed using this coating composition solution and a plain tablet composed mainly of lactose and cornstarch to evaluate the maximum spray rate, the minimum coating time, and the water vapor transmission rate of the coating composition.

The maximum spray rate in Comparative Example 1 was 7.1 g/min, and the coating time required to achieve a coating mass gain of 3% was 142 minutes. The dissolution rate in 10 minutes was 79%, and the water vapor transmission rate was 60 g/m²·day. The results are shown in Table 2.

### Comparative Example 2

7.9 parts by mass of partially saponified PVA (manufactured by JAPAN VAM & POVAL CO., LTD., PE-05JPS, saponification value: 88.2 mol%, viscosity of 4% by mass aqueous solution: 5.3 mPa·s), 18.5 parts by mass of bentonite (manufactured by KUNIMINE INDUSTRIES CO., LTD., KUNIPIA F), and 3.6 parts by mass of sorbitan monolaurate (manufactured by Wako Pure Chemical Industries, Ltd.) were added to 970.0 parts by mass of purified water with stirring using a homogenizer. The mixture was stirred for 1 hour to prepare a coating composition solution (concentration in water: 3% by mass). The coating test was performed using this coating composition solution and a plain tablet composed mainly of lactose and cornstarch to evaluate the maximum spray rate, the minimum coating time, and the water vapor transmission rate of the coating composition.

The maximum spray rate in Comparative Example 2 was 7.0 g/min, and the coating time required to achieve a coating mass gain of 3% was 144 minutes. The dissolution rate in 10 minutes was 75%, and the water vapor transmission rate was 37 g/m²·day. The results are shown in Table 2.

As is clear in Table 2, Examples 1 to 8, which used the coating composition of the present invention, showed high moisture-proofness. In addition, Examples 1 to 8 showed a high spray rate, resulting in a shortened time to achieve a predetermined coating mass gain of tablets. The high spray rate means that the adhesion between tablets and between tablets and the pan is less likely to occur. Further, Examples 1 to 8 showed a high dissolution rate in 10 minutes, indicating that the fast-dissolving property was comparable to that of ordinary fast-dissolving tablets.

In contrast, Comparative Examples 1 and 2 showed a low spray rate and failed efficient coating.

Further, Comparative Examples 1 and 2 showed a low dissolution rate in 10 minutes low and failed fast dissolution.

The above results demonstrate that the coating composition of the present invention can provide highly moisture-proof, highly productive, and fast-dissolving coating, etc.

**[Table 1]**

| | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|
| PVA(PE-05JPS) | 40.0 | 40.0 | 35.0 | 40.0 | 39.0 | 40.0 | 70.0 | 75.0 | 40.0 | 26.4 |
| Bentonite | 10.0 | 10.0 | 15.0 | 15.0 | 1.0 | 10.0 | 20.0 | 15.0 | 60.0 | 61.6 |
| PVA : Bentonite (mass ratio) | 80:20 | 80:20 | 70:30 | 73:27 | 97.5:2.5 | 80:20 | 78:22 | 83:17 | 40:60 | 30:70 |
| Sorbitan monolaurate | 0.0 | 10.0 | 0.0 | 0.0 | 0.0 | 0.0 | 0.0 | 0.0 | 0.0 | 12.0 |
| Sucrose fatty acid ester | 10.0 | 0.0 | 10.0 | 10.0 | 10.0 | 30.0 | 10.0 | 6.0 | 0.0 | 0.0 |
| Talc | 40.0 | 40.0 | 40.0 | 35.0 | 50.0 | 20.0 | 0.0 | 4.0 | 0.0 | 0.0 |
| Total | 100.0 | 100.0 | 100.0 | 100.0 | 100.0 | 100.0 | 100.0 | 100.0 | 100.0 | 100.0 |
| Aqueous solution conc. (%) | 10 | 10 | 10 | 10 | 15 | 10 | 10 | 10 | 3 | 3 |

**[Table 2]**

| Coating results | No. | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|---|
| | Maximum spray rate (g/min.) | 5.5 | 5.8 | 7.5 | 6.6 | 6.7 | 5.0 | 3.8 | 4.1 | 7.1 | 7.0 |
| | Coating time (min.) | 55 | 52 | 40 | 45 | 30 | 60 | 79 | 73 | 142 | 144 |
| Film property | Water vapor transmission rate (g/m²·day) | 53 | 54 | 36 | 40 | 80 | 50 | 58 | 80 | 60 | 37 |
| Dissolution test results | Dissolution rate in 10 min. (%) | 100 | 98 | 100 | 98 | 99 | 99 | 83 | 85 | 79 | 75 |

### INDUSTRIAL APPLICABILITY

The coating composition of the present invention can provide fast and highly productive coating and form a coating film having quite highly moisture-proofness. Therefore, the coating composition of the present invention is very useful industrially in the production of oral solid preparations.

## Claims

1. A coating composition for oral solid preparations, the coating composition comprising a polyvinyl alcohol-based polymer (a), a water-swellable clay mineral (b), and a fatty acid ester of a polyol having 3 or more hydroxy groups (c), wherein the polyvinyl alcohol-based polymer (a) and the water-swellable clay mineral (b) are present at a mass ratio of 98:2 to 65:35 in the coating composition.

2. A coating composition for oral solid preparations, the coating composition comprising a polyvinyl alcohol-based polymer (a), a water-swellable clay mineral (b), a fatty acid ester of a polyol having 3 or more hydroxy groups (c), and talc (d) .

3. The coating composition for oral solid preparations according to claim 2, wherein the polyvinyl alcohol-based polymer (a) and the water-swellable clay mineral (b) are present at a mass ratio of 98:2 to 65:35 in the coating composition.

4. The coating composition for oral solid preparations according to claim 2 or 3, wherein the talc (d) is present at 5 to 50% by mass of the whole composition.

5. The coating composition for oral solid preparations according to any one of claims 1 to 4, wherein the polyvinyl alcohol-based polymer (a) and the fatty acid ester of a polyol having 3 or more hydroxy groups (c) are present at a mass ratio of 95:5 to 50:50 in the coating composition.

6. The coating composition for oral solid preparations according to any one of claims 1 to 5, wherein the fatty acid ester of a polyol having 3 or more hydroxy groups (c) comprises at least one kind selected from a sugar fatty acid ester and a sugar alcohol fatty acid ester.

7. The coating composition for oral solid preparations according to any one of claims 1 to 6, wherein the fatty acid ester of a polyol having 3 or more hydroxy groups (c) comprises at least one kind selected from a sucrose fatty acid ester and a sorbitan fatty acid ester.

8. The coating composition for oral solid preparations according to any one of claims 1 to 7, wherein the water-swellable clay mineral (b) comprises at least one kind selected from bentonite and aluminum magnesium silicate.

9. The coating composition for oral solid preparations according to any one of claims 1 to 8, the coating composition further comprising a colorant.

10. The coating composition for oral solid preparations according to any one of claims 1 to 9, wherein a 4% by mass aqueous solution of the polyvinyl alcohol-based polymer (a) has a viscosity of 2.0 to 10.0 mPa·s measured by the method specified in JIS K6726.

11. The coating composition for oral solid preparations according to any one of claims 1 to 10, wherein a 4% by mass aqueous solution of the polyvinyl alcohol-based polymer (a) has a viscosity of 2.0 to 10.0 mPa·s measured by the method specified in JIS K6726, the water-swellable clay mineral (b) comprises a water-swellable clay mainly containing montmorillonite and the fatty acid ester of a polyol having 3 or more hydroxy groups (c) comprises at least one kind selected from a sugar fatty acid ester and a sugar alcohol fatty acid ester.

12. An oral solid preparation coated with the coating composition according to any one of claims 1 to 11.

13. A method for producing an oral solid preparation, the method comprising the step of applying or spraying an aqueous and/or water-based solution containing the coating composition according to any one of claims 1 to 11 to a solid preparation to cover a surface of the solid preparation with the coating composition.

14. Use of a composition for coating an oral solid preparation, the composition comprising a polyvinyl alcohol-based polymer (a), a water-swellable clay mineral (b), and a fatty acid ester of a polyol having 3 or more hydroxy groups (c),
wherein the polyvinyl alcohol-based polymer (a) and the water-swellable clay mineral (b) are present at a mass ratio of 98:2 to 65:35 in the composition.

15. Use of a composition for coating an oral solid preparation, the composition comprising a polyvinyl alcohol-based polymer (a), a water-swellable clay mineral (b), a fatty acid ester of a polyol having 3 or more hydroxy groups (c), and talc (d).

## Patentansprüche

1. Beschichtungszusammensetzung für orale feste Präparate, wobei die Beschichtungszusammensetzung ein Polyvinylalkohol-basiertes Polymer (a), ein in Wasser quellbares Tonmineral (b) und einen Fettsäureester eines Polyols mit 3 oder mehr Hydroxygruppen (c) umfasst, wobei das Polyvinylalkohol-basierte Polymer (a) und das in Wasser quellbare Tonmineral (b) in einem Masseverhältnis von 98:2 bis 65:35 in der Beschichtungszusammensetzung vorliegen.

2. Beschichtungszusammensetzung für orale feste Präparate, wobei die Beschichtungszusammensetzung ein Polyvinylalkohol-basiertes Polymer (a), ein in Wasser quellbares Tonmineral (b), einen Fettsäureester eines Polyols mit 3 oder mehr Hydroxygruppen (c) und Talk (d) umfasst.

3. Beschichtungszusammensetzung für orale feste Präparate nach Anspruch 2, wobei das Polyvinylalkohol-basierte Polymer (a) und das in Wasser quellbare Tonmineral (b) in einem Masseverhältnis von 98:2 bis 65:35 in der Beschichtungszusammensetzung vorliegen.

4. Beschichtungszusammensetzung für orale feste Präparate nach Anspruch 2 oder 3, wobei der Talk (d) mit 5 bis 50 Masse-% der Gesamtzusammensetzung vorliegt.

5. Beschichtungszusammensetzung für orale feste Präparate nach einem der Ansprüche 1 bis 4, wobei das Polyvinylalkohol-basierte Polymer (a) und der Fettsäureester eines Polyols mit 3 oder mehr Hydroxygruppen (c) in einem Masseverhältnis von 95:5 bis 50:50 in der Beschichtungszusammensetzung vorliegen.

6. Beschichtungszusammensetzung für orale feste Präparate nach einem der Ansprüche 1 bis 5, wobei der Fettsäureester eines Polyols mit 3 oder mehr Hydroxygruppen (c) mindestens eine Art, ausgewählt aus einem Zuckerfettsäureester und einem Zuckeralkoholfettsäureester, umfasst.

7. Beschichtungszusammensetzung für orale feste Präparate nach einem der Ansprüche 1 bis 6, wobei der Fettsäureester eines Polyols mit 3 oder mehr Hydroxygruppen (c) mindestens eine Art, ausgewählt aus einem Saccharosefettsäureester und einem Sorbitanfettsäureester, umfasst.

8. Beschichtungszusammensetzung für orale feste Präparate nach einem der Ansprüche 1 bis 7, wobei das in Wasser quellbare Tonmineral (b) mindestens eine Art, ausgewählt aus Bentonit und Aluminiummagnesiumsilicat, umfasst.

9. Beschichtungszusammensetzung für orale feste Präparate nach einem der Ansprüche 1 bis 8, wobei die Beschichtungszusammensetzung ferner ein Färbemittel umfasst.

10. Beschichtungszusammensetzung für orale feste Präparate nach einem der Ansprüche 1 bis 9, wobei eine 4 Masse-%ige wässerige Lösung des Polyvinylalkohol-basierten Polymers (a) eine Viskosität von 2,0 bis 10,0 mPa·s, gemessen mit dem in JIS K6726 spezifizierten Verfahren, hat.

11. Beschichtungszusammensetzung für orale feste Präparate nach einem der Ansprüche 1 bis 10, wobei eine 4 Masse-%ige wässerige Lösung des Polyvinylalkohol-basierten Polymers (a) eine Viskosität von 2,0 bis 10,0 mPa·s, gemessen mit dem in JIS K6726 spezifizierten Verfahren, hat, das in Wasser quellbare Tonmineral (b) einen in Wasser quellbaren Ton umfasst, der hauptsächlich Montmorillonit enthält, und der Fettsäureester eines Polyols mit 3 oder mehr Hydroxygruppen (c) mindestens eine Art, ausgewählt aus einem Zuckerfettsäureester und einem Zuckeralkoholfettsäureester, umfasst.

12. Orales festes Präparat, das mit der Beschichtungszusammensetzung nach einem der Ansprüche 1 bis 11 beschichtet ist.

13. Verfahren zur Herstellung eines oralen festen Präparats, wobei das Verfahren den Schritt des Aufbringens oder Sprühens einer wässerigen und/oder Wasser-basierten Lösung, die die Beschichtungszusammensetzung nach einem der Ansprüche 1 bis 11 enthält, auf ein festes Präparat, um eine Oberfläche des festen Präparats mit der Beschichtungszusammensetzung zu bedecken, umfasst.

14. Verwendung einer Zusammensetzung zum Beschichten eines oralen festen Präparats, wobei die Zusammensetzung ein Polyvinylalkohol-basiertes Polymer (a), ein in Wasser quellbares Tonmineral (b) und einen Fettsäureester eines Polyols mit 3 oder mehr Hydroxygruppen (c) umfasst, wobei das Polyvinylalkohol-basierte Polymer (a) und das in Wasser quellbare Tonmineral (b) in einem Masseverhältnis von 98:2 bis 65:35 in der Zusammensetzung vorliegen.

15. Verwendung einer Zusammensetzung zum Beschichten eines oralen festen Präparats, wobei die Zusammensetzung ein Polyvinylalkohol-basiertes Polymer (a), ein in Wasser quellbares Tonmineral (b), einen Fettsäureester eines Polyols mit 3 oder mehr Hydroxygruppen (c) und Talk (d) umfasst.

## Revendications

1. Composition d'enrobage pour préparations orales solides, la composition d'enrobage comprenant un polymère à base d'alcool polyvinylique (a), un minéral argileux gonflable à l'eau (b), et un ester d'acide gras d'un polyol ayant 3 groupes hydroxy ou plus (c), dans laquelle le polymère à base d'alcool polyvinylique (a) et le minéral argileux gonflable à l'eau (b) sont présents dans un rapport de masse de 98:2 à 65:35 dans la composition d'enrobage.

2. Composition d'enrobage pour préparations orales solides, la composition d'enrobage comprenant un polymère à base d'alcool polyvinylique (a), un minéral argileux gonflable à l'eau (b), un ester d'acide gras d'un polyol ayant 3 groupes hydroxy ou plus (c), et du talc (d).

3. Composition d'enrobage pour préparations orales solides selon la revendication 2, dans laquelle le polymère à base d'alcool polyvinylique (a) et le minéral argileux gonflable à l'eau (b) sont présents dans un rapport de masse de 98:2 à 65:35 dans la composition de revêtement.

4. Composition d'enrobage pour préparations orales solides selon la revendication 2 ou 3, dans laquelle le talc (d) est présent à raison de 5 à 50 % en masse de la composition entière.

5. Composition d'enrobage pour préparations orales solides selon l'une des revendications 1 à 4, dans laquelle le polymère à base d'alcool polyvinylique (a) et l'ester d'acide gras d'un polyol ayant 3 groupes hydroxy ou plus (c) sont présents dans un rapport massique de 95:5 à 50:50 dans la composition d'enrobage.

6. Composition d'enrobage pour préparations orales solides selon l'une des revendications 1 à 5, dans laquelle l'ester d'acide gras d'un polyol ayant 3 groupes hydroxy ou plus (c) comprend au moins un type choisi parmi un ester d'acide gras de sucre et un ester d'acide gras d'alcool de sucre.

7. Composition d'enrobage pour préparations orales solides selon l'une des revendications 1 à 6, dans laquelle l'ester d'acide gras d'un polyol ayant 3 groupes hydroxy ou plus (c) comprend au moins un type choisi parmi un ester d'acide gras de saccharose et un ester d'acide gras de sorbitane.

8. Composition d'enrobage pour préparations orales solides selon l'une des revendications 1 à 7, dans laquelle le minéral argileux gonflable à l'eau (b) comprend au moins une sorte choisie parmi la bentonite et le silicate d'aluminium et de magnésium.

9. Composition d'enrobage pour préparations orales solides selon l'une des revendications 1 à 8, la composition d'enrobage comprenant en outre un colorant.

10. Composition d'enrobage pour préparations orales solides selon l'une quelconque des revendications 1 à 9, dans laquelle une solution aqueuse à 4 % en masse du polymère à base d'alcool polyvinylique (a) a une viscosité de 2,0 à 10,0 mPa-s mesurée par la méthode spécifiée dans la norme JIS K6726.

11. Composition d'enrobage pour préparations orales solides orales selon l'une quelconque des revendications 1 à 10, dans laquelle une solution aqueuse à 4% en masse du polymère à base d'alcool polyvinylique (a) a une viscosité de 2,0 à 10.0 mPa-s mesurée par la méthode spécifiée dans la norme JIS K6726, le minéral argileux gonflable à l'eau (b) comprend une argile gonflable à l'eau contenant principalement de la montmorillonite et l'ester d'acide gras d'un polyol ayant 3 groupes hydroxy ou plus (c) comprend au moins un type choisi parmi un ester d'acide gras de sucre et un ester d'acide gras d'alcool de sucre.

12. Préparation solide orale enduite de la composition d'enrobage selon l'une des revendications 1 à 11.

13. Procédé de production d'une préparation solide orale, le procédé comprenant l'étape d'application ou de pulvérisation d'une solution aqueuse et/ou à base d'eau contenant la composition de revêtement selon l'une des revendications 1 à 11 sur une préparation solide pour couvrir une surface de la préparation solide avec la composition de revêtement.

14. Utilisation d'une composition pour enrober une préparation solide orale, la composition comprenant un polymère à base d'alcool polyvinylique (a), un minéral argileux gonflable à l'eau (b), et un ester d'acide gras d'un polyol ayant 3 groupes hydroxy ou plus (c), dans laquelle le polymère à base d'alcool polyvinylique (a) et le minéral argileux gonflable à l'eau (b) sont présents dans un rapport de masse de 98:2 à 65:35 dans la composition.

15. Utilisation d'une composition pour enrober une préparation orale solide, la composition comprend un polymère à base d'alcool polyvinylique (a), un minéral argileux gonflable à l'eau (b), un ester d'acide gras d'un polyol ayant 3 groupes hydroxy ou plus (c), et du talc (d).
